# EUROPEAN PATENT APPLICATION

(11) **EP 2 390 312 A1**
(43) Date of publication of application: **30.11.2011**
(21) Application number: 11177996.3
(22) Date of filing: 29.11.2006
(51) Int. Cl.: C12N 5/00, C12N 5/02, A61K 48/00

(54) **Methods of improving stem cell homing and engraftment**

(30) Priority: 29.11.2005 US 289004
(62) Divisional of application: 06821601.9
(71) Applicant: Gamida Cell Ltd., 95484 Jerusalem (IL)
(72) Inventor: Peled, Tony, 90805 Mevasseret Zion (IL)
(74) Representative: Dennemeyer & Associates S.A.

(57) **Abstract**

The present invention relates to a method of enhancing cell homing and engraftment potential, the method comprises *ex-vivo* or *in vitro* subjecting a population of cells to an amount of nicotinamide for a period of time sufficient for enhancing cell homing and engraftment potential, the method is further characterized by at least one of the following:
(i) wherein said amount of nicotinamide and said period of time are selected sufficient to down regulate CD26 expression by cells of said population of cells but not for stem and/or progenitor cell expansion;
(ii) said population of cells does not include, mononuclear cells, early liver progenitor cells, committed progenitor cells, non-hematopoietic stem and progenitor cells, or embryonic stem and progenitor cells;
(iii) said subjecting is in the absence of nutrients;
(iv) said subjecting is in the absence of a cytokine;
(v) said subjecting is in the absence of FLT-3 ligand;
(vii) said subjecting is in the absence of stem cell factor (SCF);
(vii) said subjecting is in the absence of granulocyte colony stimulating factor (GCSF);
(viii) said subjecting is in the absence of an early acting cytokine; and
(ix) said subjecting is in the absence of a late acting cytokine;
and wherein said population of cells does not include a hematopoietic stem and/or progenitor cell population.

## Description

### FIELD AND BACKGROUND OF THE INVENTION

The present invention relates to methods for improving homing, retention and engraftment efficiency of transplanted cells.

Bone marrow transplantation (BMT) is a clinical procedure in which pluripotent hematopoietic cells obtained from the bone marrow are transplanted into a patient. BMT is the treatment of choice in several hematological disorders, including malignancies, Severe Combined Immune Deficiencies (SCIDs), congenitally or genetically determined hematopoietic abnormalities, anemia, aplastic anemia, leukemia and osteopetrosis.

Primitive or pluripotent hematopoietic stem cells usually reside in the bone marrow, although cord blood is another functional source of transplantable hematopoietic stem/progenitor cells (Gluckman, E., et al 1989 N. Engl. J. Med. 321:1174). All of these primitive hematopoietic cells may be identified by their surface CD34 antigen. Hematopoietic stem cells differentiate along one of two major pathways - either into lymphoid stem cells or myeloid stem cells. Both further differentiate into progenitor cells for each type of mature blood cell. These progenitor cells have lost the capacity for self-renewal and are committed to a given cell lineage. Thus, lymphoid stem cells differentiate into T or B progenitor and myeloid stem cells differentiate into progenitor cells for erythrocytes, neutrophils, eosinophils, basophils, monocytes, mast cells, and platelets.

Under steady state conditions, the majority of hematopoietic stem and progenitor cells reside in the bone marrow and only a few of these cells are detectable in peripheral blood. However, stem cells may be mobilized into the peripheral blood by treatment with myelosuppressive agents and/or certain hematopoietic growth factors. Studies have demonstrated that peripheral blood stem cells (PBSC) infused in a host exhibit enhanced potential for engraftment as compared to bone marrow-derived stem and progenitor cells. Thus, PBSC mobilized by chemotherapy, hematopoietic growth factors or a combination of these modalities are currently used in both autologous and non-autologous transplantation settings [Anderlini, P. and Korbling, M. (1997) Stem. Cells 15, 9-17]. In the case of non-autologous transplantation, the donors of stem cells are healthy individuals and the procedure for mobilization of stem cells into the blood stream has to be achieved with minimal discomfort. In this case, stem cells mobilization with hematopoietic growth factors is preferred to mobilization with antiblastic drugs (i.e. cyclophosphamide).

In addition to stem and progenitor cells, more differentiated cells can be used for transplantation, for treatment of diseases or conditions of specific organs or tissues characterized by cell dysfunction or cell death. For many such diseases current medical therapies or surgical procedures are either inadequate or nonexistent. Cellular therapy can replace or augment existing tissue to provide restorative therapy for these conditions. Exemplary cell types suitable for transplantation include: neural tissue derived cells, hepatocytes, myocytes, retinal cells, endocrine cells, melanocytes, keratinocytes, and chondrocytes. It has been shown in both animal models and in human studies that engraftment of transplanted cells can successfully reestablish tissue function. Thus, neurons can be transplanted, for example, for Parkinson's Disease and other neurodegenerative disease. Muscle cells, such as myoblasts, can be transplanted for, for example, treatment of ischemic cardiac myopathy. Islet cells can be transplanted to treat diabetes and/or other insulin- and glucagon-related disease or conditions. Differentiated blood cells, such as lymphocytes and dendritic cells, can also be transplanted, for example, for adoptive immunotherapy with NK cells.

However, studies have shown that the majority of transplanted cells, such as hepatocytes and neural cells, are cleared from the body following transplantation, and do not localize to target organs or tissues (De Roos et al Transplantation 1997;63:513-18; Gagandeep et al, Gene Therapy 1999;6:729-36). Efforts to improve homing, retention and engraftment of transplanted cells, such as treatment of hepatocytes with Con A before implantation (Ito et al, Muscle Nerve 1998;21:291-7) have been only marginally effective. Thus, efforts have been directed to methods for pooling and storage of the freshly prepared cells (see, for example, US Patent Nos. 6,713,245 and 6,821,779 to Koopmans et al), in order to provide greater numbers of cells for transplantation.

Following transplantation, cells must migrate towards their target tissues. Chemoattractants, such as certain of the cytokines (CXCL1-CXCL16, and CCL1-CCL-27) aid in steering the cells towards their objective. Stromal cell-derived factor 1α (SDF-1a), also termed CXCL12, is a powerful chemoattractant of CD34 ⁺ cells, including hematopoietic stem cells and neural stem cells (Aiuti J. Exp. Med. 1997;185:111-120) and is widely expressed in many tissues during development (McGrath Dev. Biol. 1999;213:442-456) and adulthood (Imai Br. J. Haematol. 1999;106:905-911). It also chemoattracts non-stem cells such as T lymphocytes. CXC chemokine receptor 4 (CXCR4) is the cognate receptor for SDF-1α and is expressed on stem cells. Recent studies have implicated SDF-1α/CXCR4 as a pathway that activates stem cells molecular programs and homing during injury (Jaime Imitola et al., Proc Natl Acad Sci U S A. 2004 December 28; 101(52): 18117-18122).

CD26/dipeptidylpeptidase IV (DPPIV) a membrane-bound extracellular peptidase that cleaves dipeptides such as SDF-1α from the N terminus of polypeptide chains after a proline or an alanine, is a non-lineage-specific antigen whose expression in hematopoietic and other cells is regulated by differentiation and activation. Proteolytic cleavage of chemokines has implications with respect to the ability of cells to be attracted and/or activated by chemokines(Baggiolini, M.. 1998, Nature 392:565).

Several functional studies allude to the role CD26/DPPIV plays in migration and mobilization of T-cells and hematopoietic cells [Shioda et al. (1998) Proc. Natl. Acad. Sci. USA 95:6331]. Inhibition of endogenous CD26/DPPIV activity on CD34⁺ cells was shown to enhance the chemotactic response of these cells to SDF-1α (Christopherson KW 2nd, et al., Science. 2004 Aug 13;305(5686):1000- 1003; Christopherson KW 2nd, J Immunol. 2002 Dec 15;169(12):7000-7008), while N-terminal-truncation of SDF-1α with DPPIV results in failure to induce the migration of CD34⁺ cord blood cells.

Nicotinamide (NA), the amide form of niacin (vitamin B3), is a base-exchange substrate and a potent inhibitor of NAD(+)-dependent enzymes endowed with mono-and poly-ADP-ribosyltransferase activities. ADP-ribosylation is implicated in the modification of a diverse array of biological processes (Corda D, Di Girolamo M. 2003;22(9):1953-1958; Rankin PW, et al., J Biol Chem. 1989;264:4312-4317; Banasik M. et al., J Biol Chem. 1992;267:1569- 1575; Ueda K, Hayaishi O, Annu Rev Biochem. 1985;54:73-100; Smith S. Trends Biochem Sci. 2001;26:174-179;Virág L, Szabó C. Pharm. Reviews. 2002;54:375-429).

The endogenous ADP-ribosyl transferases responsible for mono- or poly-ADP-ribosylation reactions modify molecules involved in cell signaling, such as core histones (de la Cruz X, Lois S, et al., Bioessays. 2005;27(2):164-75), the alpha-subunit of heterotrimeric GTP-binding (G) proteins, the small GTPase Rho, monomeric actin and elongation factor 2 (EF-2). These post-translational modifications lead to activation or inactivation of cell functions modulated by these proteins (Lupi R, et al., J Biol Chem. 2000;275:9418-9424; Lupi R, et al. Biochem J. 2002:367:1-7; Yau L, et al., Eur. J. Biochem. 2003;270:101-110).

U.S. Pat. Appl. 2004/0247574 teaches the use of CD26 inhibitors for improving engraftment efficiency of stem cell transplants by both improving stem cell homing to bone marrow and by increasing the number of mobilized donor stem cells. It does not teach down-regulation of CD26 surface expression but rather down regulation of CD26 catalytic activity. Specifically, U.S. Pat. Appl. 2004/0247574 does not teach the use of nicotinamide for down-regulating CD26 surface expression.

PCT Application IL03/00064 discloses the use of nicotinamide, and other inhibitors of CD38, for the inhibition of differentiation in *ex-vivo* expanding stem and progenitor cells. However, PCT IL03/00064 does not teach administration of nicotinamide for enhancing homing, retention and engraftment of cells, or administration of nicotinamide to stem and progenitor cells for short intervals of 3 days or less, administration of nicotinamide to non-stem and non-progenitor (i.e. committed) cell populations or the administration of nicotinamide without provision of conditions for cellular proliferation.

It is therefore the object of this invention to overcome the drawbacks described in the currently available treatments and provide compositions and methods for the enhancement of cell migration, retention and homing potential of transplanted cells.

### SUMMARY OF THE INVENTION

According to one aspect of the present invention there is provided a method of enhancing cell engraftment potential, the method comprising *ex-vivo* or *in-vitro* subjecting a population of cells to an amount of nicotinamide for a period of time sufficient to enhance cell homing and engraftment potential, wherein the method is further characterized by at least one of the following:
(i) wherein said population of cells is a hematopoietic stem and/or progenitor cell population, and said period of time is selected insufficient for stem cell expansion, or under conditions insufficient for stem and/or progenitor cell expansion;
(ii) wherein said amount of nicotinamide and said period of time are selected sufficient to down regulate CD26 expression by cells of said population of cells but not for stem and/or progenitor cell expansion;
(iii) said population of cells does not include hematopoietic cells, hematopoietic stem cells, mononuclear cells, early liver progenitor cells, committed progenitor cells, non-hematopoietic stem and progenitor cells, or embryonic stem and progenitor cells;
(iv) said subjecting is in the absence of nutrients;
(v) said subjecting is in the absence of a cytokine;
(vi) said subjecting is in the absence of FLT-3 ligand;
(vii) said subjecting is in the absence of stem cell factor (SCF);
(viii) said subjecting is in the absence of granulocyte colony stimulating factor (GCSF);
(ix) said subjecting is in the absence of an early acting cytokine; and
(x) said subjecting is in the absence of a late acting cytokine.

According to another aspect of the present invention there is provided a method of transplanting cells in a subject, the method comprising: (a) ex-vivo subjecting a population of cells comprising the cells to an amount of nicotinamide for a period of time sufficient to enhance homing and engraftment in said cells; the method further characterized by at least one of the following:
(i) wherein said population of cells is a hematopoietic stem and/or progenitor cell population, and said period of time is selected insufficient for stem cell expansion, or under conditions insufficient for stem and/or progenitor cell expansion;
(ii) wherein said amount of nicotinamide and said period of time are selected sufficient to down regulate CD26 expression by cells of said population of cells but not for stem and/or progenitor cell expansion;
(iii) said population of cells does not include hematopoietic cells, hematopoietic stem cells, mononuclear cells, early liver progenitor cells, committed progenitor cells, non-hematopoietic stem and progenitor cells, or embryonic stem and progenitor cells;
(iv) said subjecting is in the absence of nutrients;
(v) said subjecting is in the absence of a cytokine;
(vi) said subjecting is in the absence of FLT-3 ligand;
(vii) said subjecting is in the absence of stem cell factor (SCF);
(viii) said subjecting is in the absence of granulocyte colony stimulating factor (GCSF);
(ix) said subjecting is in the absence of an early acting cytokine;
(x) said subjecting is in the absence of a late acting cytokine; and subsequently

### (b) transplanting the cells in a subject in need thereof.

According to still further features in the described preferred embodiments the subject is a human subject.

According to still further features in the described preferred embodiments the nicotinamide is selected from the group consisting of nicotinamide, a nicotinamide analog, a nicotinamide metabolite, a nicotinamide analog metabolite and derivatives thereof.

According to still further features in the described preferred embodiments the population of cells is derived from an organ selected from the group consisting of a muscle, skin, a bone, a lymph organ, a pancreas, a liver, a gallbladder, a kidney, a digestive tract organ, a respiratory tract organ, a reproductive organ, a urinary tract organ, a blood-associated organ, a thymus, a spleen, a nervous system organ.

According to still further features in the described preferred embodiments the population of cells does not include hematopoietic cells, mononuclear cells, early liver progenitor cells, committed progenitor cells, non-hematopoietic stem and progenitor cells, or embryonic stem and progenitor cells.

According to still further features in the described preferred embodiments, the population of cells comprises stem cells.

According to still further features in the described preferred embodiments the stem cells are derived from a source selected from the group consisting of hematopoietic cells, umbilical cord blood cells, mobilized peripheral blood cells, bone marrow cells and embryonic stem and/or progenitor cells.

According to still further features in the described preferred embodiments the stem cells are derived from bone marrow or peripheral blood.

According to still further features in the described preferred embodiments the stem cells are derived from neonatal umbilical cord blood.

According to still further features in the described preferred embodiments the stem cells are derived from a mononuclear cell fraction.

According to still further features in the described preferred embodiments the stem cells are enriched for hematopoietic stem cells.

According to still further features in the described preferred embodiments the methods of enhancing stem cell engraftment potential and transplanting further comprising the step of selecting the population of cells enriched for hematopoietic stem cells prior to, concomitant with or following the step of ex-vivo subjecting.

According to still further features in the described preferred embodiments the selecting is effected via CD34

According to still further features in the described preferred embodiments the methods of enhancing stem cell engraftment potential and transplanting, further comprising the step of selecting the population of cells enriched for early hematopoietic stem cells prior to, concomitant with or following the step of ex-vivo subjecting.

According to still further features in the described preferred embodiments the selecting is effected via CD 133.

According to still further features in the described preferred embodiments the selecting is effected via CD34/CD38.

According to still further features in the described preferred embodiments the period of time is between 1 and 18 weeks.

According to still further features in the described preferred embodiments the period of time is between 1 and 7 days.

According to still further features in the described preferred embodiments the period of time is between 2 and 4 days.

According to still further features in the described preferred embodiments the period of time is between 12-30 hours.

According to still further features in the described preferred embodiments the period of time does not exceed 72 hours.

According to still further features in the described preferred embodiments said population of cells is a hematopoietic stem and progenitor cell population, and said period of time is selected insufficient for stem cell expansion.

According to still further features in the described preferred embodiments said population of cells is a hematopoietic stem and progenitor cell population, and said subjecting is performed under conditions insufficient for stem cell expansion.

According to still further features in the described preferred embodiments said conditions insufficient for stem cell expansion are selected from the group consisting of absence of nutrients, absence of late acting cytokines and absence of early acting cytokines.

According to still further features in the described preferred embodiments said period of time is sufficient to downregulate expression of CD26 on the cells, but insufficient for cell proliferation.

According to still further features in the described preferred embodiments a concentration of the nicotinamide is 0.01-60 mg/ml.

According to still further features in the described preferred embodiments the effective amount of nicotinamide is 1.0-40 mg/kg body weight.

According to still further features in the described preferred embodiments the effective amount of nicotinamide is 10-20 mg/kg body weight.

According to still another aspect of the present invention there is provided a cell population comprising the cells characterized by enhanced homing and/or engraftment according to the above methods.

According to an additional aspect of the present invention there is provided a pharmaceutical composition comprising as an active ingredient the cell population and a pharmaceutically acceptable carrier.

According to yet an additional aspect of the present invention there is provided use of nicotinamide for the manufacture of a medicament identified for improving stem cell engraftment and/or homing.

The present invention successfully addresses the shortcomings of the presently known configurations by providing methods for enhancing stem cell mobilization and migration, both prior to and following stem cell transplantation.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

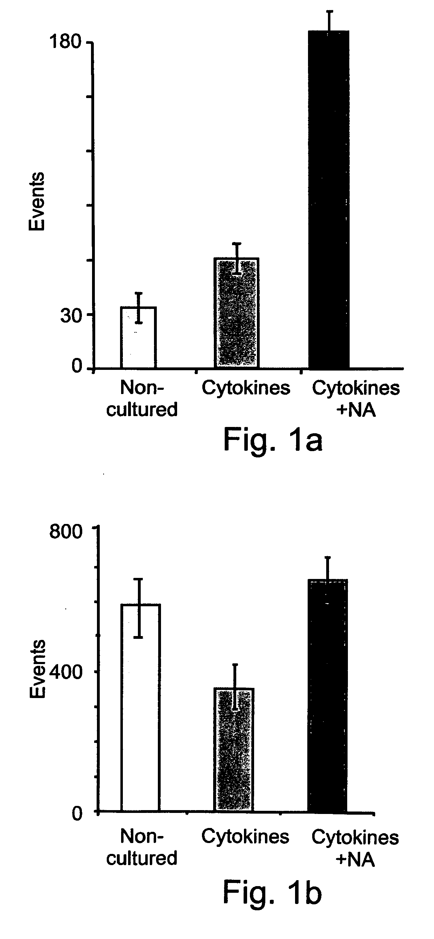

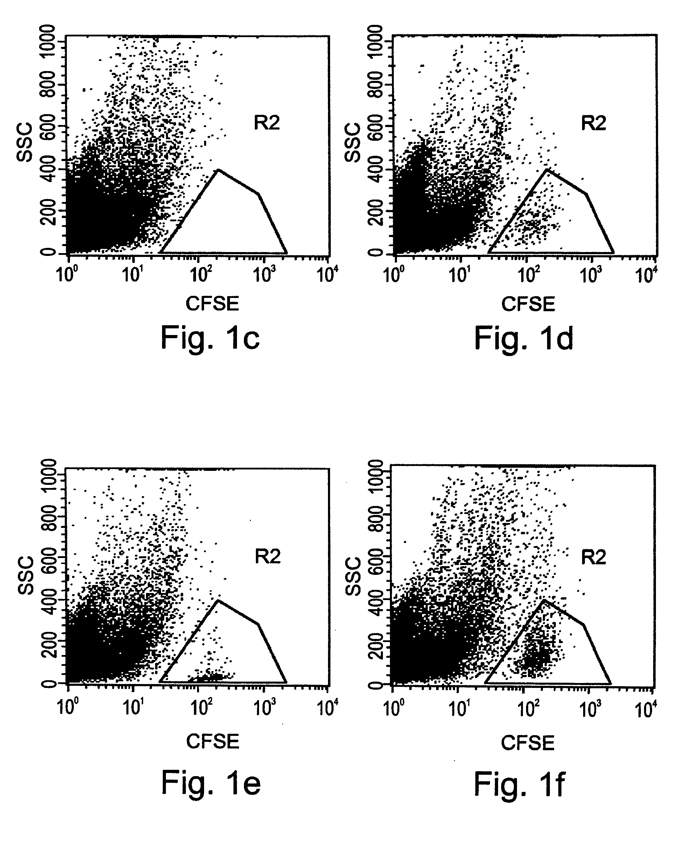

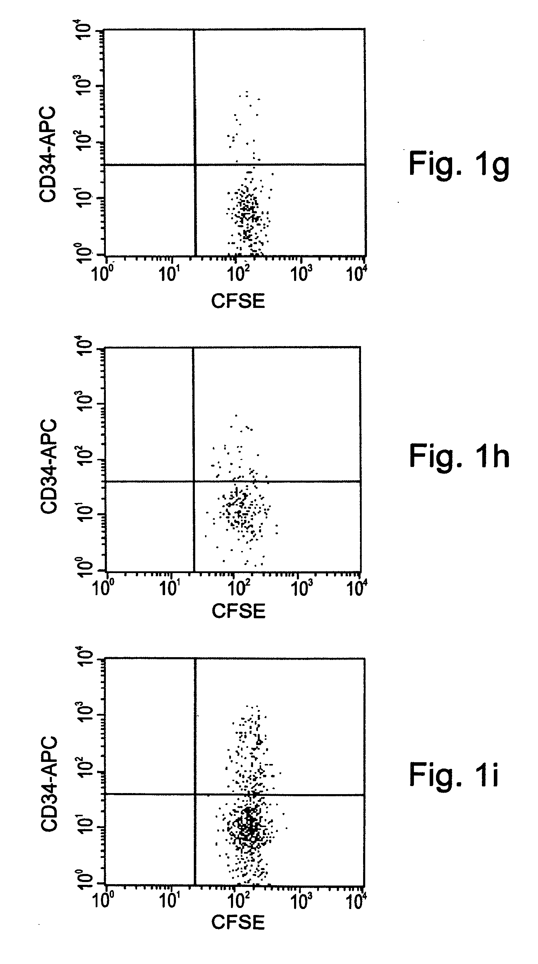

FIGs. 1a-1i are a graphic representation of flow cytometry analysis of the effect of nicotinamide on homing of hematopoietic stem cells into bone marrow of NOD/SCID mice. Non-cultured mononuclear cells or their entire progeny following 3-week expansion with cytokines and nicotinamide (cytokines+NA), or cytokines alone (cytokines) were labeled with CFSE and infused into sublethally irradiated NOD/SCID mice (10 x 10⁶ cells/mouse for the non-cultured group containing 5x10⁴ CD34+ cells and the total progeny of 5x10⁴ CD34+ cells following 3-week expansion with or without nicotinamide; 20 x10⁶ cells/mouse containing 180 x10⁴ CD34+ cells). Figure 1a is a histogram of the results of flow cytometry of recipient's bone marrow cells showing homing of CFSE+/CD34+ cells after transplantation. Figure 1b is a histogram of the results of flow cytometry of recipient's bone marrow cells showing homing of total CFSE+ cells after transplantation. Homing of human cells is presented as number of positive events (cytometry) per 100,000 BM cells analyzed. Each bar represents the average ± SE of 3 independent experiments (6-7 mice/experimental group). Representative flow cytometry analysis of bone marrow cells from non-injected mice (Figure 1c) and mice injected with non-cultured cells (Figures 1d and 1g), cells cultured with cytokines (Figures 1e and 1h) and cells cultured with cytokines and nicotinamide (Figures If and 1i) are shown. Total human cells that home to the BM are gated (see area R2) based on low side scatter (y axis) and log fluorescence distribution of CFSC expression (x axis) (Figures 1c-1f). The bright fluorescence of CFSE was sufficient to separate labeled human cells from unlabeled murine cells by at least 1 log. Cells gated in R2 were then analyzed for CFSE (x axis) and CD34-APC (y axis) (Figures 1g-1i). The upper and lower right quadrants represent total human cells while the upper right quadrant represents the human CD34+ cells that home to the BM;

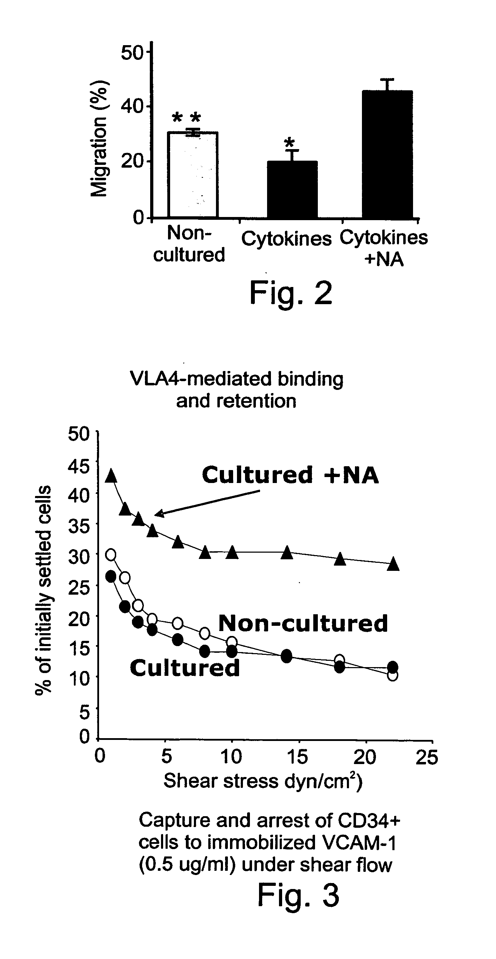

FIG. 2 is a histogram showing the effect of nicotinamide on *in-vitro* migration of hematopoietic cells. CXCL12 (100ng/ml)-induced transwell migration of the purified CD34+ cells either before (non-cultured) or after 3-week culture with cytokines and nicotinamide (cytokines+NA) or cytokines alone (cytokines) (n=3, *p<0.02, **p=0.05) was measured as described hereinbelow. Note the enhanced migration of cells cultured in the presence of nicotinamide;
FIG. 3 is a graph showing the effect of nicotinamide on VLA4-mediated binding of cells to immobilized adhesion molecules under shear flow. CD34+ cells cultured as described in Figs. 1 and 2 hereinabove were assayed for capture and arrest under shear stress, on immobilized VCAM-1 adsorbed as 10 µl dots on polystyrene. Cell settling events, and arrest were analyzed under perfusion (shear stress) by video photography. Assayed cell populations were cells before culture (non-cultured, open circles), cells cultured with cytokines, as described in Fig. 2 (cultured, closed circles), and cells cultured with cytokines and nicotinamide (cultured+NA, closed triangles). Note the significant and consistent effect of nicotinamide on adhesion molecule-mediated binding;

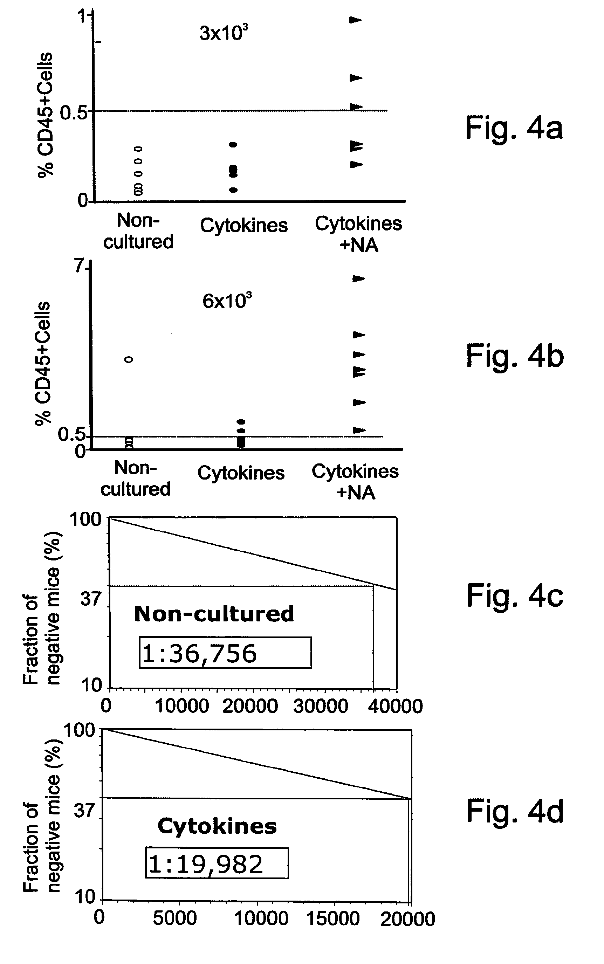

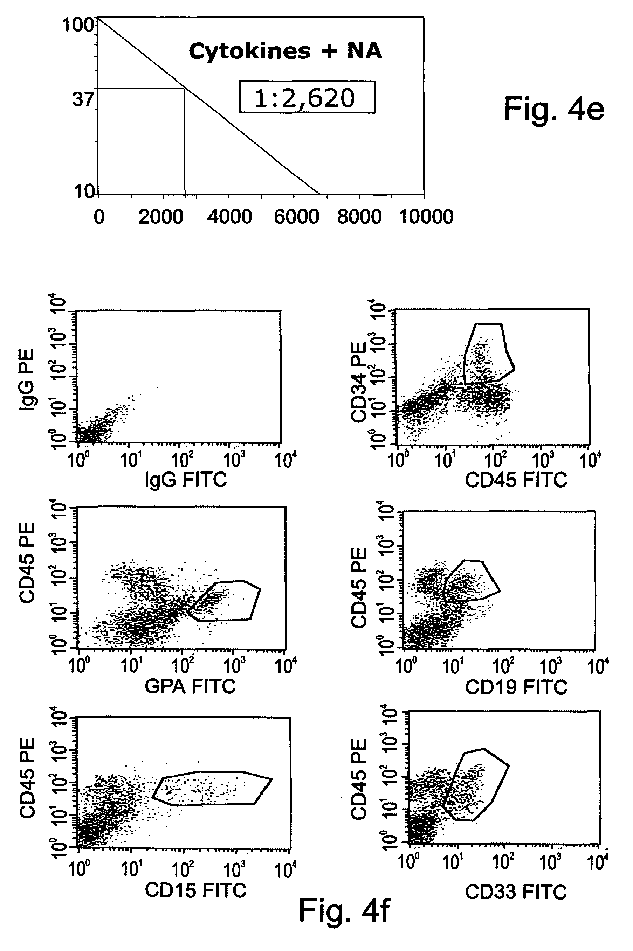

FIGs. 4a-4f is a graphic representation of the effect of nicotinamide on homing and engraftment of human hematopoietic cells transplanted into NOD/SCID mice. Figures 4a and 4b show the percentage of human (CD45+) cells in the cell populations before transplantation: non-cultured CD34⁺ cells (non-cultured, gray ovals), the entire progeny of cultures following 3-week exposure to cytokines alone (cytokines alone, closed ovals), or cytokines and nicotinamide (cytokines+NA, arrows). The percent of engraftment 4 weeks post transplantation was determined by flow cytometry of human CD45 cells in the NOD/SCID marrow (y-axis). The numbers of SCID repopulating cells (SRC) were calculated by plotting the engraftment frequencies at each dose. The resultant curves indicates the estimated frequency of SRCs within non-cultured CD34+ cells (Figure 4c), culture with cytokines (Figure 4d) or cytokines and nicotinamide (Figure 4e). The number shown in each box indicates the calculated frequency of SRCs using the maximum likelihood estimator. Figure 4f shows the immunophenotype of engrafted human cells in representative mice transplanted with the progeny of 12x10³ CD34+ cells cultured for 3-weeks with nicotinamide, as determined by FACS. Mouse bone marrow cells were dually stained with FITC-conjugated anti-CD45 (human) and antibodies to human differentiation markers as indicated. Percentages of dual positive cells are shown within each quadrant. Note the greater than 7 fold enhancement of engraftment in the nicotinamide-treated mice (Fig. 4e), as compared with mice treated with cytokines alone (Fig. 4d);

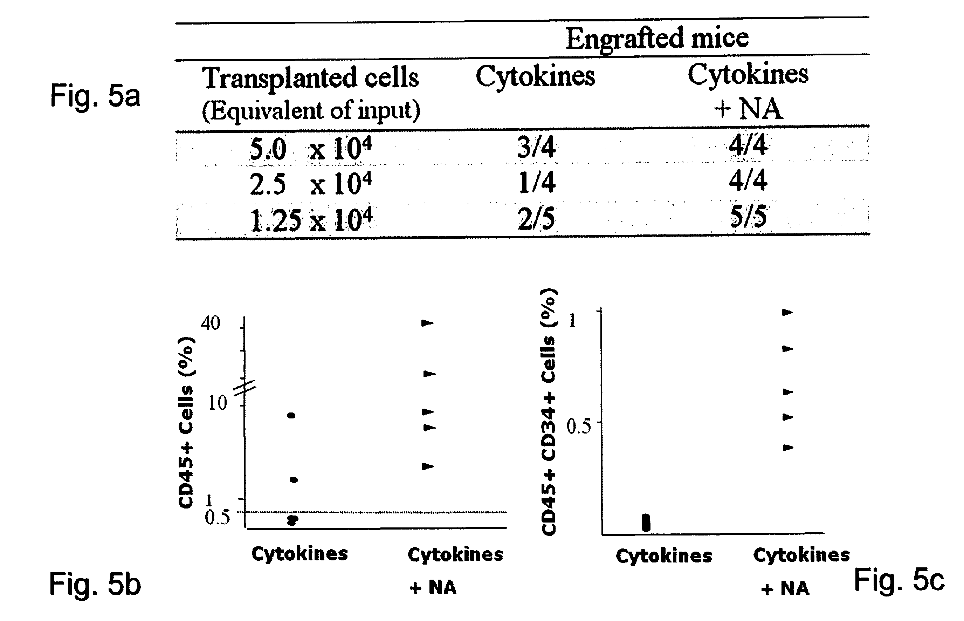

FIGs. 5a-5c are a graphic representation of the effect of nicotinamide (NA) on the engraftment potential of cells cultured under differentiation-promoting conditions. Cultures were initiated with purified cord blood-derived CD34+ cells in medium supplemented with SCF, TPO, IL-6 and FLT3, 50 ng/ml each, and IL-3, 20 ng/ml, with (cytokines+NA, arrows) or without (cytokines, ovals) 10 mM nicotinamide. After 3 weeks, cells were harvested, and transplanted into SCID mice as indicated. Mice were transplanted with 1.25 - 5 x 10⁴ CD34+ cells or with their progeny following expansion. Mice were sacrificed 4 weeks later and bone marrow cells were analyzed by FACS for the presence of CD34+ (human progenitor) and CD45+ (human) cells. Mice were scored as engrafted when the number of human (CD45+) cells constituted ≥0.5% of the marrow population. Figure 4a shows the numbers of positively engrafted mice per total number of transplanted mice, for each of the doses of transplanted cells (5.0X10⁴; 2.5X10⁴; and 1.25X10⁴ cells). Figures 4b and 4c show the percentage of total human (CD45+)(Fig. 4b) cells and human progenitor (CD45+CD34+)(Fig. 4c) cells in the bone marrow of mice transplanted with cells derived from culture initiated with 1.25 x 10⁴ CD34+ cells. Note the enhanced engraftment of both total human, and human progenitor cells resulting from nicotinamide treatment.

### DESCRIPTION OF THE PREFERRED EMBODIMENTS

The present invention is of methods of improving homing and engraftment of transplantable cells.

The principles and operation of the present invention may be better understood with reference to the accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Successful blood and marrow transplantations, both autologous and allogenic, require the infusion of a sufficient number of hematopoietic stem cells capable of homing to the marrow cavity and regenerating a full array of hematopoietic cell lineages in a timely fashion. Recruitment of stem cells from the marrow into the blood is termed mobilization, or, more commonly stem cell mobilization. It is well established that enhancement of stem cell mobilization and/or homing will result in successful stem cell transplantation.

SDF-α chemoattracts hematopoietic stem and progenitor cells (HSCs/HPCs) and is thought to play a crucial role in the mobilization of HSCs/HPCs from the bone marrow as well as in stem cell homing.

CD26 is a widely distributed 110 kDa cell-surface glycoprotein with known dipeptidyl peptidase IV (DPPIV) activity in its extracellular domain capable of cleaving N-terminal dipeptides from polypeptides with either proline or alanine residues in the penultimate position. CD26 inhibits SDF activity by cleaving the latter at its position-2 proline, thereby inhibiting its stem cell mobilizing/homing functions.

U.S. Pat. Appl. 2004/0247574 teaches the use of CD26 inhibitors for improving engraftment efficiency of stem cell transplants by both improving stem cell homing to bone marrow and by increasing the number of mobilized donor stem cells. It does not teach down-regulation of CD26 surface expression but rather down regulation of CD26 catalytic activity. Specifically, U.S. Pat. Appl. 2004/0247574 does not teach the use of nicotinamide for down-regulating CD26 surface expression.

While reducing the present invention to practice, the present inventor uncovered that nicotinamide can be successfully used to down-regulate cell-surface expression of CD26, enhance the expression and function of adhesion and integrin molecules, increase induced transplantable cell migration, and significantly improve homing and engraftment of transplantable cells *in-vivo.*

As is demonstrated hereinbelow and in the Examples section which follows, the present inventor showed that short-term incubation of cells with nicotinamide is sufficient to down-regulate CD26 expression. A significant reduction in CD26-expressing CD34+ and AC133+ cells was observed following as few as 20 hours of incubation with nicotinamide.

Of greater significance, nicotinamide was shown effective in enhancing the functionality of molecules critical to the process of cell binding and arrest. Indeed, evaluation of cell migration potential *in-vitro* following nicotinamide exposure showed that nicotinamide both enhanced CXCL12-inducible migration and VLA-4-mediated binding and retention on VCAM-1 in transplantable cells cultured with nicotinamide(see Example 3 below). Since the processes of cell migration are mediated by "recognition" pairs such as VLA-4 and VCAM-1 in a wide variety of cells, this surprising finding indicates that nicotinamide, and nicotinamide derivatives and analogs, can be effective in enhancing binding and retention, critical to the ability of transplanted cells to successfully home, engraft and repopulate host tissues, for cells of diverse origins and stages of differentiation.

Actual *in-vivo* transplantation experiments with nicotinamide-treated cells provided conclusive evidence for the effect of nicotinamide on cell engraftment and homing potential. Example 2 below shows that exposure of mononuclear cells to nicotinamide prior to transplantation into NOD/SCID mice increased the homing to bone marrow sixfold over identical cells cultured without nicotinamide. Yet further, Example 4 below shows that at clearly sub-optimal doses of cells, while control cultured cells failed to cause repopulation of bone marrow in NOD/SCID mice, transplantation of nicotinamide-treated cells resulted in a high degree of engraftment and successful repopulation.

Taken together these results suggest a novel role for nicotinamide in cell homing and engraftment and as such in cell transplantation.

Thus, according to one aspect of the present invention there is provided a method of enhancing cell homing and engraftment potential, the method comprising ex-vivo or *in vitro* subjecting a population of cells to an amount of nicotinamide for a period of time sufficient for enhancing cell homing and engraftment potential.

As used herein the phrase "enhancing cell engraftment potential" refers to an improvement in efficiency, quality or rapidity of cell transplantation which may result from improved homing to the target tissue, improved adhesion, reduced rejection and the like. Methods for assessing cell engraftment potential include, for example, cell migration and other *in vitro* techniques, and histological, immunological and/or radiological assessment of tissues and organs from actual *in-vivo* transplantation, as described in detail hereinbelow. A self renewal potential of the stem cells can be determined in-vitro by long term colony formation (LTC-CFUc), or by *in-vivo* engraftment in the SCID-Hu mouse model. The SCID-Hu mouse model employs C.B-17 scid/scid (SCID) mice transplanted with human fetal thymus and liver tissue or fetal BM tissue and provides an appropriate model for the evaluation of putative human hematopoietic stem cells. Because of the reconstitution of the SCID mice with human fetal tissue, the model affords the proliferation of stem cells, in this case human hematopoietic stem cells to proliferate, and function in the hematopoietic microenvironment of human origin. Mice are typically irradiated, then delivered stem cells into the grafts, and reconstitution is measured by any number of methods, including FACS and immunohistochemistry of repopulated organs (Humeau L., et al Blood (1997) 90:3496; also see Materials and Experimental Methods below).

As used herein the term *"ex-vivo"* refers to a process in which cells are removed from a living organism and are propagated outside the organism (e.g., in a test tube).

As used herein, the term *"in-vitro"* refers to a process in which cells originating from a cell line or lines (such as NTera2 neural cells, embryonic cell lines, etc.) maintained in the laboratory, are manipulated outside of an organism. Such cell lines are often immortalized cells.

As used herein the phrase "population of cells" refers to a homogeneous or heterogeneous isolated population of cells which comprise cell populations suitable for transplantation. In a preferred embodiment, at least a portion of the population of cells of this aspect of the present invention expresses CD26 or VLA-4 on the cell-surface.

As used herein, the phrase "stem cells" refers both to the earliest renewable cell population responsible for generating cell mass in a tissue or body and the very early progenitor cells, which are somewhat more differentiated, yet are not committed and can readily revert to become a part of the earliest renewable cell population.

As used herein, the phrases "non-stem", "non-progenitor" and "committed cells" refer to cells at various stages of differentiation, which generally no longer retain the ability to revert to become a part of a renewable cell population. Methods of ex-vivo culturing stem, progenitor, and non-stem, non-progenitor committed cells are well known in the art of cell culturing. To this effect, see for example, the text book "Culture of Animal Cells - A Manual of Basic Technique" by Freshney, Wiley-Liss, N. Y. (1994), Third Edition, the teachings of which are hereby incorporated by reference.

The population of cells of the present invention may be from an autologous or non-autologous donor (allogeneic or xenogeneic).

In a preferred embodiment, the cells for transplantation are stem and/or progenitor cells, and the source of the stem cell population is an unfractionated mononuclear cell preparation, not having been enriched for CD34+ or other hematopoietic stem cells. In another embodiment, the stem cells are identified by stem cell markers such as CD34+, CD34+/CD38-, CD133+, CD34+/Lin-, and other stem cell markers known in the art. In yet another embodiment, the source of the stem cell population are stem cells having been enriched for hematopoietic stem cells by selection according to stem cell markers.

For example, stem cells of the present invention may be derived from a source selected from the group consisting of hematopoietic cells, umbilical cord blood cells, and mobilized peripheral blood cells.

As used herein "nicotinamide" refers to nicotinamide as well as to products that are derived from nicotinamide, analogs thereof and metabolites of nicotinamide or nicotinamide analogs, such as, for example, NAD, NADH and NADPH.

As used herein, the phrase "nicotinamide analog" refers to any molecule that is known to act similarly to nicotinamide. Representative examples of nicotinamide analogs include, without limitation, benzamide, nicotinethioamide (the thiol analog of nicotinamide), nicotinic acid and α-amino-3-indolepropionic acid.

As used herein the term "subject" refers to a mammalian subject, preferably a human subject.

The phrase "a nicotinamide or a nicotinamide analog derivative" refers to any structural derivative of nicotinamide itself or of an analog of nicotinamide. Examples of such derivatives include, without limitation, substituted benzamides, substituted nicotinamides and nicotinethioamides and N-substituted nicotinamides and nicotinthioamides.

Additionally or alternatively, stem cell mobilization may be effected prior to harvest of cells for stem cell transplantation using mobilizing agents which are well known in the art. Generally, the mobilization process is initiated by stress-induced activation of neutrophils and osteoclasts by chemotherapy and repeated stimulation with cytokines such as granulocyte colony-stimulating factor (G-CSF), resulting in shedding and release of membrane-bound stem cell factor (SCF), proliferation of progenitor cells, as well as activation and/or degradation of adhesion molecules. Mobilizing agents which may be used in accordance with the present invention include, but are not limited to, DNA damaging agents, single chemotherapeutic agents (e.g., cyclophosphamide), combined chemotherapy regimens [e.g., iphosphamide, carboplatin and etoposide (ICE) and methylprednisolone, ara-c, and cisplatin (ESHAP)], cytokines such as G-CSF, GM-CSF, SCF, FLT-3 ligand, and chemokines such as IL-8, MIP-1α, Groβ, and SDF-1. The mode of administration, as well as the time frame needed to achieve mobilization and the types of cells mobilized depend on the molecules used. For example, G-CSF is usually administered daily as a dose of 5-10 µg/gk for 5-10 days, alone or after therapy. The adjustment of the mobilization regimen is done by the physician and reviewed in Cottker-Fox et al. (2003) Hematology 419-437.

Methods of preparation of cells for transplantation are well known in the art. For preparation of non-stem cells, cells can be obtained from donor tissue by dissociation of individual cells from the connecting extracellular matrix of the tissue. Tissue from a particular region is removed using a sterile procedure, and the cells are dissociated-using any method known in the art including treatment with enzymes such as trypsin, collagenase, DNAse and the like, or by using physical methods of dissociation such as with a blunt instrument.

Cells prepared for transplantation can be maintained in a physiological solution, or cultured in suspension or on a fixed substrate. Suitable culture media capable of supporting cells include HEM, DMEM, RPMI, F-12, and the like. If required, the medium can contain supplements required for cellular metabolism such as glutamine and other amino acids, vitamins, minerals and useful proteins such as transferrin, and the like. The medium may also contain antibiotics to prevent contamination with yeast, bacteria, and fungi, such as penicillin, streptomycin, gentamicin, and the like. If cells are to be cultured, conditions should be close to physiological conditions (preferably, a pH of about 6 to about 8, and a temperature of about 30° C. to about 40° C.). The culture medium can be optionally supplemented with at least one proliferation-inducing growth factor, such as EGF, amphiregulin, acidic fibroblast growth factor (aFGF or FGF-1), basic fibroblast growth factor (bFGF or FGF-2), transforming growth factor alpha (TGF-alpha), cytokines such as G-CSF, GM-CSF, SCF, FLT-3 ligand, and/or chemokines such as IL-8, MIP-1α, Groβ, and SDF-1, and combinations thereof. In addition to proliferation-inducing growth factors, other growth factors may be added to the culture medium, including NGF, platelet-derived growth factor (PDGF), thyrotropin releasing hormone (TRH), and the like.

Selection and enrichment of specific cell types can be performed, such as separation of hepatocytes from liver tissue, separation of neurons from glial cells, or isolation of islet cells from pancreatic tissue, by morphological, physical, immunohistochemical (FACS) or other means. Fresh or cultured cell preparations can be cryopreserved until they are needed by any method known in the art. The cells can be suspended in an isotonic solution, preferably a cell culture medium, containing a particular cryopreservant. Such cryopreservants include dimethyl sulfoxide (DMSO), glycerol, and the like. Further methods for preparation and storage of cells for transplantation are known in the art, and disclosed in detail in, for example, the Handbook of Transplantation (Kipshidze and Serruys, eds. London, UK, 2004).

Methods of preparation of stem cells are well known in the art, commonly selecting cells expressing one or more stem cell markers such as CD34, CD133, etc, or lacking markers of differentiated cells. Selection is usually by FACS, or immunomagnetic separation, but can also be by nucleic acid methods such as PCR (see Materials and Experimental Methods hereinbelow). Embryonic stem cells and methods of their retrieval are well known in the art and are described, for example, in Trounson AO (Reprod Fertil Dev (2001) 13: 523), Roach ML (Methods Mol Biol (2002) 185: 1), and Smith AG (Annu Rev Cell Dev Biol (2001) 17:435). Adult stem cells are stem cells, which are derived from tissues of adults and are also well known in the art. Methods of isolating or enriching for adult stem cells are described in, for example, Miraglia, S. et al. (1997) Blood 90: 5013, Uchida, N. et al. (2000) Proc. Natl. Acad. Sci. USA 97: 14720, Simmons, P.J. et al. (1991) Blood 78: 55, Prockop DJ (Cytotherapy (2001) 3: 393), Bohmer RM (Fetal Diagn Ther (2002) 17: 83) and Rowley SD et al. (Bone Marrow Transplant (1998) 21: 1253), Stem Cell Biology Daniel R. Marshak (Editor) Richard L. Gardner (Editor), Publisher: Cold Spring Harbor Laboratory Press, (2001) and Hematopoietic Stem Cell Transplantation. Anthony D. Ho (Editor) Richard Champlin (Editor), Publisher: Marcel Dekker (2000).

It will be appreciated that nicotinamide can enhance engraftment and homing potential in a wide variety of cell types. For example, nicotinamide can down-regulate surface expression of CD26, and enhance functionality of VLA-4, CXCR-2 or other adhesion molecules from any cell-type expressing same and since these molecules are widely expressed in cell populations of diverse origin, the population of cells of this aspect of the present invention may comprise unselected cell populations, such as crude cell preparations from tissue, or mononuclear stem and/or progenitor cells, as well as more homogenous populations of selected cell types.

As used herein, the phrase "hematopoietic mononuclear cells" refers to the entire repertoire of white blood cells present in a blood sample, usually hematopoietic mononuclear cells which comprise a major fraction of hematopoietic committed cells and a minor fraction of hematopoietic stem and progenitor cells. In a healthy human being, the white blood cells comprise a mixture of hematopoietic lineages committed and differentiated cells (typically over 99 % of the mononuclear cells are lineages committed cells) including, for example: Lineage committed progenitor cells CD34⁺CD33⁺ (myeloid committed cells), CD34⁺CD3⁺ (lymphoid committed cells) CD34⁺CD41⁺ (megakaryocytic committed cells) and differentiated cells - CD34⁻ CD33⁺ (myeloids, such as granulocytes and monocytes), CD34⁻CD3⁺, CD34⁻CD19⁺ (T and B cells, respectively), CD34⁻CD41⁺ (megakaryocytes), and hematopoietic stem and early progenitor cells such as CD34⁺Lineage negative (Lin⁻), CD34-Lineage negative CD34⁺CD38⁻ (typically less than 1 %).

Hematopoietic mononuclear cells are typically obtained from a blood sample by applying the blood sample onto a Ficoll-Hypaque layer and collecting, following density-cushion centrifugation, the interface layer present between the Ficoll-Hypaque and the blood serum, which interface layer essentially entirely consists of the white blood cells present in the blood sample.

Presently, hematopoietic stem cells can be obtained by further enrichment of the hematopoietic mononuclear cells obtained by differential density centrifugation as described above. This further enrichment process is typically performed by immuno-separation such as immunomagnetic-separation or FACS and results in a cell fraction that is enriched for hematopoietic stem cells (for detailed description of enrichment of hematopoietic stem cells, see Materials and Experimental Procedures in the Examples section hereinbelow).

Regardless of the origin of cells employed and their composition, once the cells are obtained, they are subjected to (contacted with) an amount of nicotinamide for a period of time sufficient to enhance engraftment and homing of the cells following transplantation. Such a period of time may be brief, or lengthier, as required. In one preferred embodiment, the contacting is for a period of time sufficient to down-regulate CD26 surface expression. In another preferred embodiment, the cells are hematopoietic stem cells, and the contacting is for a period of time insufficient for stem cell proliferation (also referred to as expansion) while sufficient to down-regulate CD26 surface expression. In yet another embodiment, the contacting is for a period of time sufficient to increase functionality of VLA-4, CXCR2 and other adhesion and/or integrin molecules.

Methods of determining protein cell-surface expression are well known in the art. Examples include immunological methods, such as, FACS analysis (see Examples section) as well as biochemical methods (cell-surface labeling, e.g., radioactive, fluorescence, avidin-biotin).

Methods of assaying cell-proliferation are well known in the art (such as MTT, thymidine incorporation, FACS). It will be appreciated that cell doubling rate may also be derived from the literature.

Depending on the cell type, and the intended use thereof, cells may be ex-vivo subjected to nicotinamide for long-term contact, i.e. periods of weeks or more, and cells may even be stored in contact with nicotinamide prior to use for transplantation. Further, according to certain embodiments, short-term exposure is desirable. Long-term contacting can be for between 1 and 18 weeks, preferably between 3 and 9 weeks, more preferably between 2 and 5 weeks, most preferably between 2 and 3 weeks. Short term contacting can be for 1 to 2 weeks, preferably one week or less, more preferably between 1-5 days.

While reducing the present invention to practice, it was uncovered that 20 hours exposure of hematopoietic stem cells to nicotinamide was sufficient to effect a reduction in CD26 expression, crucial to cell homing and engraftment, although insufficient to allow for stem cell expansion or proliferation to take place. Thus, according to one embodiment of the present invention, cells are ex-vivo subjected to nicotinamide for a period of time not exceeding a few days, preferably 30 hours, more preferably 1-30 hours, even more preferably 5-30 hours, even more preferably 10-30 hours. In another preferred embodiment, the cells are stem and/or progenitor cells, and the duration of exposure to nicotinamide is selected insufficient for stem cell expansion, or under conditions insufficient for stem cell expansion, such as absence of cytokines, absence of nutrients, sub-optimal temperature, etc.

Nicotinamide is preferably provided in a final concentration of 0.01-60 mg/ml, preferably 1-40 mg/ml, more preferably 5-30 mg/ml, and most preferably 10-20 mg/ml. The selection of culture medium and medium supplements, depends on the cells and their intended use.

In one preferred embodiment cells subjected to nicotinamide can be used for transplantation to a subject in need thereof following nicotinamide exposure for the predetermined period of time, without further ex-vivo expansion. It will be appreciated that exposure to nicotinamide can be performed on cells that have received additional ex-vivo treatment, such as expansion, selection, genetic modification, etc. well known in the art, including prior ex-vivo exposure to nicotinamide, and preferably closely preceding the use thereof for engraftment.

Following exposure to nicotinamide, the cells may then be transplanted in (administered to) a subject in need thereof. The following summarizes some clinical applications which may be addressed according to the teachings of the present invention.

Hematopoietic cell transplantation: Transplantation of hematopoietic cells has become the treatment of choice for a variety of inherited or malignant diseases. While early transplantation procedures utilized the entire bone marrow (BM) population, recently, more defined populations, enriched for stem cells (CD34⁺ cells) have been used [Van Epps Blood Cells 20:411, (1994)]. In addition to the marrow, such cells could be derived from other sources such as peripheral blood (PB) and neonatal umbilical cord blood (CB) [Emerson Blood 87:3082 (1996)]. Compared to BM, transplantation with PB cells shortens the period of pancytopenia and reduces the risks of infection and bleeding [Brugger N Engl J Med 333:283, 1995; Williams Blood 87:1687, (1996); Zimmerman J Heamatotherapy 5:247, (1996)].

An additional advantage of using PB for transplantation is its accessibility. The limiting factor for PB transplantation is the low number of circulating pluripotent stem/progenitor cells.

To obtain enough PB-derived stem cells for transplantation, these cells are "harvested" by repeated leukophoresis following their mobilization from the marrow into the circulation by treatment with chemotherapy and cytokines [Brugger N Engl J Med 333:283, 1995; Williams Blood 87:1687, (1996)]. Such treatment is obviously not suitable for normal donors.

The use of ex-vivo expanded stem cells for transplantation has the following advantages [Koller Blood 82:378, (1993); Lebkowski Blood Cells 20:404, (1994)]:
It reduces the volume of blood required for reconstitution of an adult hematopoietic system and may obviate the need for mobilization and leukophoresis [Brugger N Engl J Med 333:283, 1995].
It enables storage of small number of PB or CB stem cells for potential future use.

In the case of autologous transplantation of recipients with malignancies, contaminating tumor cells in autologous infusion often contribute to the recurrence of the disease [Brugger N Engl J Med 333:283, 1995]. Selecting and expanding CD34⁺ stem cells will reduce the load of tumor cells in the final transplant.

The cultures provide a significant depletion of T lymphocytes, which may be useful in the allogeneic transplant setting for reducing graft-versus-host disease.

Clinical studies indicate that transplantation of ex-vivo expanded cells derived from a small number of PB CD34⁺ cells can restore hematopoiesis in recipients treated with high doses of chemotherapy, although the results do not yet allow firm conclusions about long term in-vivo hematopoietic capabilities of these cultured cells [Brugger N Engl J Med 333:283, 1995; Williams Blood 87:1687, (1996)].

For successful transplantation, shortening of the duration of the cytopenic phase, as well as long-term engraftment, is crucial. Inclusion of intermediate and late progenitor cells in the transplant could accelerate the production of donor-derived mature cells thereby shortening the cytopenic phase. It is important, therefore, that ex-vivo expanded cells include, in addition to stem and/or progenitor cells subjected to nicotinamide as described hereinabove, more differentiated cells in order to optimize short-term recovery and long-term restoration of hematopoiesis. Inclusion of expanded intermediate and late committed cells, especially those committed to the neutrophilic and megakaryocytic lineages, concomitant with the expanded stem and/or progenitor cells, should serve this purpose [Sandstrom Blood 86:958, (1995)].

Such cultures may be useful in restoring hematopoiesis in recipients with completely ablated bone marrow, as well as in providing a supportive measure for shortening recipient bone marrow recovery following conventional radio- or chemotherapies.

Tissue regeneration: Stem cell populations of the present invention, can be used for the promotion of tissue regeneration. Transplantation of stem cells, has great promise for benefits in regenerative medicine, reconstructive surgery, tissue engineering, regenerating new tissues and naturally healing diseased or injured organs (for review see Czyz et al, Biol Chem, 2003;384:1391-40, Sylvester et al Arch Surg 2004;139:93-99). Further, neurons and supporting glial cells have been used for transplantation in treatment of Huntington's disease (US Patent No. 6,524, 865 to Freed et al) and pancreatic islet cells are being used for transplantation for type I and type II diabetes (see, for example, US Patents 6,326,201, to Fung et al; and 7,045,349 to Benedict et al). Muscle, and muscle derived cells, are being investigated for clinical use, and have shown promising results when transplanted into injured heart tissue, bone tissue and articular structures (see US Patent No. 6,866,842 to Chancellor, et al). Thus, according to one aspect of the instant invention, the cells for engraftment or transplantation can be derived from an organ selected from the group consisting of a muscle, skin, a bone, a lymph organ, a pancreas, a liver, a gallbladder, a kidney, a digestive tract organ, a respiratory tract organ, a reproductive organ, a urinary tract organ, a blood-associated organ, a thymus, a spleen and a nervous system organ. Examples of cells which can be prepared for implantation by the methods of the present invention include primary cultures as well as established cell lines. Examples of these include, but are not limited to pancreatic islet cells, human foreskin fibroblasts, beta cell insulomas, NT2 cells, embryonic cells, embryonic stem cells, hepatocytes, dopamine secreting ventral mesencephalon cells, neuroblastoid cells, adrenal medulla cells, T-cells combinations of these, and the like. As can be seen from this partial list, cells of all types, including dermal, neural, blood, organ, muscle, glandular, bone, digestive, reproductive and immune system cells, as well as cells of all species of origin, can be prepared successfully by this method.

Recent reports have demonstrated the capability of transplanted or transfused stem cells to enhance regeneration in non-homologous tissue, other than that which the stem cells were derived. For example, enhanced myogenesis and angiogenesis in infarcted myocardium have been observed following infusion of bone marrow stem cells (Tse et al, Lancet 2003, 361:47-79, Jackson et al J Clin Invest 2001;107:1395-402, Orlic et al Nature 2001;410:701-5; Lee et al, Cell Cycle 2005;4:861-64, Nagaya et al Am J Heart Circ Phys 2004;287:H2670-76). Other studies have shown bone marrow, endothelial and skeletal muscle stem cells to be beneficial in ischemic renal injury (Togel et al, AJP Renal Phys 2005;289:F31-F42 and Arriero, et al, AMJ Ren Phys 2004;287:F621-27).

Gene therapy: For successful long-term gene therapy, a high frequency of genetically modified cells with transgenes stably integrated within their genome, is an obligatory requirement. In BM tissue, for example, while the majority of cells are cycling progenitors and precursors, stem cells constitute only a small fraction of the cell population and most of them are in a quiescent, non-cycling state. Viral-based (e.g., retroviral) vectors require active cell division for integration of the transgene into the host genome. Therefore, gene transfer into fresh stem cells is highly inefficient. The ability to store and process a selected population of cells *ex-vivo,* and enhance their homing and engraftment potential would provide for an increased probability of the successful use of genetically modified cell transplantation [Palmiter Proc Natl Acad Sci USA 91(4): 1219-1223, (1994)].

Adoptive immunotherapy: Ex-vivo-expanded, defined lymphoid subpopulations have been studied and used for adoptive immunotherapy of various malignancies, immunodeficiencies, viral and genetic diseases [Freedman Nature Medicine 2: 46, (1996); Heslop Nature Medicine 2: 551, (1996); Protti Cancer Res 56: 1210, (1996)].

The treatment enhances the required immune response or replaces deficient functions. This approach was pioneered clinically by Rosenberg et al. [Rosenberg J Natl Cancer Inst. 85: 622, 1993] using a large number of autologous and also allogeneic ex-vivo expanded non-specific killer T cells, and subsequently ex-vivo expanded specific tumor infiltrating lymphocytes.

Functionally active, antigen-presenting cells could be grown from a starting population of CD34⁺ PB cells in cytokine-supported cultures, as well. These cells can present soluble protein antigens to autologous T cells in-vitro and, thus, offer new prospects for the immunotherapy of minimal residual disease after high dose chemotherapy. Ex-vivo expansion of antigen-presenting dendritic cells has been studied as well, and is an additional promising application of the currently proposed technology [Bernhard Cancer Res 10: 99, (1995); Fisch Eur J Immunol 26: 595, (1996); Siena Expt Hematol 23:1463, (1996)].

Additional examples for ex-vivo applications:
Additional applications of non-stem, differentiated cells, as well as stem and progenitor cell treatment with nicotinamide include skin regeneration, hepatic regeneration, muscle regeneration, stimulation of bone growth for applications in osteoporosis and transplantation of chondrocytes/chondroblasts and/or synovial cells for treatment of articular and arthritic disorders.

According to one aspect of the present invention, the ex-vivo contacting of cell populations with nicotinamide, according to the features described hereinabove, can be utilized for preparing a population of stem or non-stem cells *ex-vivo* or *in-vitro* for implanting the cells in an organ of a subject in need thereof.

Cells of the present invention may be transplanted by means of direct injection into an organ, injection into the bloodstream, intraperitoneal injection, etc. Suitable methods of transplantation can be determined by monitoring the homing and engraftment of the implanted cells to the desired organ, the expression of desired organ-specific genes or markers, and the function of the derived organ of the subject. In the pancreas, for example, maintenance of euglycemia, secretion of insulin and/or C peptide can be a measure of the restoration of function to a diabetic host animal following cell replacement therapy as disclosed hereinbelow. In the liver, for example, albumin synthesis can be monitored.

Cell populations of the present invention can be provided per se, along with the culture medium containing same, isolated from the culture medium, and combined with a pharmaceutically acceptable carrier as well as with additional agents which may promote cell engraftment and/or organ function (e.g., immunosuppressing agents, antibiotics, growth factor). Hence, cell populations of the invention can be administered in a pharmaceutically acceptable carrier or diluent, such as sterile saline and aqueous buffer solutions. The use of such carriers and diluents is well known in the art.

Compositions of the present invention may, if desired, be presented in a pack or dispenser device, such as an FDA-approved kit, which may contain one or more unit dosage forms containing the active ingredient (e.g., cells). The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the invention formulated in a pharmaceutically acceptable carrier may also be prepared, placed in an appropriate container, and labeled for treatment of an indicated condition, as further detailed above.

The cells prepared according to the methods of the present invention can be administered to the subject per se, or in a pharmaceutical composition where it is mixed with suitable carriers or excipients.

As used herein, a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Hereinafter, the phrases "physiologically acceptable carrier" and "pharmaceutically acceptable carrier," which may be used interchangeably, refer to a carrier or a diluent that does not cause significant irritation to an organism and does not abrogate the biological activity and properties of the administered compound. An adjuvant is included under these phrases.

Herein, the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils, and polyethylene glycols.

Techniques for formulation and administration of drugs may be found in the latest edition of "Remington's Pharmaceutical Sciences," Mack Publishing Co., Easton, PA, which is herein fully incorporated by reference.

Suitable routes of administration may, for example, include oral, rectal, transmucosal, especially transnasal, intestinal, or parenteral delivery, including intramuscular, subcutaneous, and intramedullary injections, as well as intrathecal, direct intraventricular, intravenous, intraperitoneal, intranasal, or intraocular injections.

Alternately, one may administer the pharmaceutical composition in a local rather than systemic manner, for example, via injection of the pharmaceutical composition directly into a tissue region of a patient.

Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

Pharmaceutical compositions suitable for use in the context of the present invention include compositions wherein the active ingredients are contained in an amount effective to achieve the intended purpose. More specifically, a "therapeutically effective amount" means an amount of active ingredients (e.g., a nucleic acid construct) effective to prevent, alleviate, or ameliorate symptoms of a disorder (e.g., ischemia) or prolong the survival of the subject being treated.

Determination of a therapeutically effective amount is well within the capability of those skilled in the art, especially in light of the detailed disclosure provided herein.

For any preparation used in the methods of the invention, the dosage or the therapeutically effective amount can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration, and dosage can be chosen by the individual physician in view of the patient's condition. (See, e.g., Fingl, E. et al. (1975), "The Pharmacological Basis of Therapeutics," Ch. 1, p.1.)

Depending on the severity and responsiveness of the condition to be treated, dosing can be of a single or a plurality of administrations, with course of treatment lasting from several days to several weeks, or until cure is effected or diminution of the disease state is achieved.

The amount of a composition to be administered will, of course, be dependent on the subject being treated, the severity of the affliction, the manner of administration, the judgment of the prescribing physician, etc.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### MATERIALS AND EXPERIMENTAL PROCEDURES

***Cord blood samples** -* Cells were obtained from umbilical cord blood after normal full-term delivery (informed consent was given). Samples were collected and frozen according to Rubinstein et al. [Rubinstein et al. Proc Natl Acad Sci U S A. 1995; 92 (22):10119-10122.] within 24 h postpartum. Prior to use, the cells were thawed in Dextran buffer (Sigma, St. Louis, MO, USA) containing 2.5 % human serum albumin (HAS, Bayer Corp. Elkhart, IN, USA), layered on a Ficoll-Hypaque gradient (1.077 g/mL; Sigma), and centrifuged at 800 x g for 30 min. The mononuclear cells in the interface layer were collected and washed three times in phosphate-buffered saline (PBS, Biological Industries) containing 0.5 % HSA. To purify the CD34+ cells, the mononuclear cell fraction was subjected to two cycles of immunomagnetic bead separation, using a "MiniMACS CD34 progenitor cell isolation kit" (Miltenyi Biotec Bergisch, Gladbach, Germany), according to manufacturer's instructions. The purity of the CD34+ population thus obtained was 95-98 %, as evaluated by flow cytometry.

***FACS analysis of CD34+ cells** -* Percentages of progenitor cells positive for CD26 were determined by staining of CD34+ cells with anti-CD26 FITC purchased from Becton Dickinson.

***Ex vivo expansion-*** Purified CD34+ cells were cultured in culture bags (American Fluoroseal Co., Gaithersburg, MD) at 1x10⁴ cells/ml in MEMα medium, 10% fetal calf serum (FCS) and cytokines: Thrombopoietin (TPO), interleukin-6 (IL-6), FLT-3 ligand and stem cell factor (SCF), each at a final concentration of 50 ng/ml (Perpo Tech, Inc., Rocky Hill, NJ), with or without 5 mM nicotinamide (NA) (Sigma Aldrich, Milwaukee, WI) and incubated at 37° C in a humidified atmosphere of 5% CO₂ in air. Preliminary studies conducted with various concentrations of NA (1 - 10 mM), indicated that in combination with the 4 cytokines, 5 mM was the optimal NA concentration (data not shown). Until week 3, the cultures were topped weekly with the same volume of fresh medium and then weekly demi-depopulated. Cell counting, colony forming unit (CFUc) assay and immunophenotype analysis were performed as described hereinbelow.

***Immunophenotyping of CD34+ cells-*** MiniMACS re-isolated CD34+ cells were washed with PBS solution containing 1% BSA and double stained (at 4°C for 30 min) with PE-conjugated anti-CD34 and FITC-conjugated antibodies to CXCR4, VLA-4 (Chemicon Intnl, Inc. Temecula, CA, USA), LFA-1 (IQ product), CD38, or with a mixture of FITC-conjugated antibodies against differentiation antigens (CD38, CD33, CD14, CD15, CD3, CD61, CD19) for determination of CD34+Lin-cells. (Antibodies to CD34, CD38 and CD61 were purchased from DAKO Glostrup, Corp, Carpenteria, CA, USA, while the others were from Becton Dickinson and Co, San Jose, CA, USA). The cells were then washed in the above buffer and analyzed, using a FACScalibur® flow cytometer (Becton Dickinson and Co., San Jose, CA). Emission of 10⁴ cells was measured using logarithmic amplification, and analyzed using CellQuest software (Becton Dickinson and Co, San Jose, CA, USA). The FACS analysis results are presented as percentages of CD34+ cells. The absolute number of CD34+CD38- and CD34+Lin- cells was calculated from the total number of CD34+ cells in culture.

***CFSE labeling-*** Non-cultured or cultured cells were washed and resuspended at less than 10⁷ cells/mL in serum-free medium. CFSE (Molecular Probes, Inc., Eugene, OR, USA) was added at a final concentration of 5 µg/ml, and the cells were incubated for 10 minutes at 37°C. Uptake of the dye was stopped by the addition of 10% FCS. After labeling, cells were washed three times in PBS supplemented with 10% FCS and analyzed by flow cytometry for fluorescence intensity, then injected intravenously into sublethally irradiated NOD/SCID mice (10-20 million cells per mouse).

***In Vitro Migration Assay-*** RPMI plus 10% FCS (0.6 ml) containing 100 ng/ml CXCL12 (R&D Systems) was put into the lower chamber of a Costar 24-well "transwell" culture plate (Coming, Inc, Coming, NY). Cells (2 x10⁵) in 100-µl medium were introduced into the upper chamber, over a porous membrane (pore size, 5 µm). After 4 hours, cells were collected from both chambers and counted by flow cytometry (FACSsort, Becton Dickinson and Co, San Jose, CA, USA). Spontaneous migration control migration was performed without CXCL12 in the lower chamber.

***In vivo analysis of homing-*** NOD/SCID mice (8-10 week old) (Harlan Ltd., Israel) were sub-lethally irradiated (at 375cGy at 67cGy/min) and 24 hours later inoculated via the tail vein with either CFSE-labeled cultured, or non-cultured CB cells. Mice were sacrificed at 24 hours post injection and bone marrow samples were obtained by flushing their femurs and tibias with IMDM at 4°C. Homing of human cells was detected by flow cytometry via visualization of CFSE-stained cells over a background of unlabeled murine cells. The bright fluorescence of CFSE was sufficient to separate labeled human cells from unlabeled murine cells by at least 1 log. To quantify homing of human progenitor cells, bone marrow cells were stained with APC-conjugated antihuman CD34 monoclonal antibodies and CFSE⁺CD34⁺ (human progenitor) cells were enumerated. For each sample 100,000 events were recorded and analyzed.

***Transplantation of human CD34⁺ cells into NOD*/*SCID mice-*** NOD/SCID mice were bred and maintained in sterile intra-ventilated cages (Techniplast, Bugugiatte, Italy). Eight-week-old mice were sub-lethally irradiated as described above. Mice were then inoculated via the tail vein with fresh purified CB-derived CD34⁺ cells or their entire progeny following 3-weeks in culture. To avoid donor variability, CB-derived CD34⁺ cells from several units were pooled and used for expansion cultures as well as group injection. Mice were sacrificed at week 4, and marrow samples were obtained by flushing their femurs and tibias with IMDM at 4°C. Flow cytometric analysis of NOD/SCID marrow cells was performed as described hereinabove, using monoclonal antibodies against human leukocyte differentiation antigens to identify human cell engraftment.

***Quantification of SCID repopulating cells (SRCs)-*** The frequencies of SRCs were quantified by a limiting dilution analysis and applying Poisson statistics to the single-hit model as described previously. Mice were scored as positively engrafted if 0.5% of their marrow cells expressed human CD45. The frequencies of SRCs and statistical comparison between individual populations were calculated by maximum likelihood estimator using L-Calc. Software (StemCell Technologies, Vancouver, BC).

***Shear Flow Experiments-*** Soluble purified seven-domain human VCAM-1, sVCAM-1 was mixed in coating medium (PBS buffered with 20 mM sodium bicarbonate pH, 8.5) with a fixed amount of carrier (2 µg/ml HSA) and adsorbed as 10-µl spots on polystyrene plates (Becton Dickinson and Co, San Jose, CA, USA) for 2 hours at 37°C, alone or with the indicated amounts of intact or heat-inactivated chemokines. Plates were washed and blocked with HSA (20 mg/ml). VCAM-1 site densities were assessed using ¹²⁵I-labeled anti-VCAM-1 mAb, 4B9. Cell monolayers of non-cultured cells and cells following culturing with or without nicotinamide and VCAM-1/chemokine-coated substrates were assembled as the lower surface of the flow chamber (260-µm gap) and extensively washed with binding medium. The flow chamber was mounted on the stage of an inverted phase contrast microscope (Diaphot 300; Nikon Europe BV, Badhoevedorp, The Netherlands). All flow experiments were conducted at 37°C. Cells were perfused at 10⁶cells/ml through the chamber at desired flow rate generated with an automated syringe pump. The entire duration of cell perfusions were recorded on a videotape with a long integration LIS-700 CCD video camera (Applitech Rigicam, Israel) and a Time Lapse SVHS-Video recorder (AG-6730; Panasonic, Japan). All cellular interactions with the adhesive substrates were determined by manually tracking the motions of individual cells along 0.9-mm field paths for 1 min. Cellular interactions with VCAM-1-bearing surfaces were >95% α4 integrin dependent. In each experiment all events were normalized to a constant cell population flowing in immediate proximity with the substrate. Frequency of each category of tethers was expressed in percentage of units (event x cell⁻¹ x 10²); 1% unit measured at 0.5, 1, and 1.5 dyn/cm² corresponded to tethering rate of 1.5 x 10⁻³, 3 x 10⁻³, and 4.5 x 10⁻³ events x cell⁻¹ mm⁻¹ s⁻¹, respectively, expressed as the mean ± range or SD.

***Statistics-*** The non-parametric Wilcoxon Rank Test was applied for testing differences between the study groups. All the tests applied were two-tailed, and a p value of ≤ 5% was considered statistically significant. The data were analyzed using SAS software (SAS Institute, Cary, NC).

### EXPERIMENTAL RESULTS

### EXAMPLE 1

### Nicotinamide down-regulates CD26/dipeptidylpeptidase IV expression on CD34+ cells

The effect of short-term incubation with nicotinamide on HSC CD26 membranal expression was addressed by FACS analysis.

Freshly purified CD34+ cells were FACS analyzed for the expression of CD26 (T-0) then incubated +/- Nicotinamide 5mM for 20 hours (T-20 hours) and FACS analyzed again. As demonstrated in a duplicate experiment summarized in Table 1 below, following 20 hours incubation (T-20) in the presence of Nicotinamide, CD26 expression was significantly reduced by the presence of Nicotinamide (2-3 fold reduction) as compared to cells maintained for 20 hours in the absence of Nicotinamide as well as to freshly purified CD34+ cells (T-0).

**Table 1**

| | CD26+ cells (%) | | |
|---|---|---|---|
| | T-0 | T-20 | |
| | | Control | + Nicotinamide |
| Exp. 1 | 8.5 | 5 | 2.7 |
| Exp. 2 | 10 | 12 | 6 |

### EXAMPLE 2

### Nicotinamide increases bone marrow homing of cultured cells

Reduced engraftment efficacy of cultured cells has been attributed, at least in part, to a defect in their homing ability relative to non-cultured cells (Szilvassy, S.J., et al, Blood, 2000;95:2829-37). To evaluate the effect of nicotinamide on the homing of cultured cells, NOD/SCID mice were transplanted with either 10x10⁶ non-cultured mononuclear cells (MNC), containing 5x10⁴ CD34+ cells (0.5% CD34+ cells), or with their total progeny following 3-weeks in culture with cytokines, with or without nicotinamide, each transplantation containing 180x10⁴ CD34+ cells. Prior to transplantation, the cells were labeled with CFSE. Twenty-four hours post transplantation total CFSE-labeled cells and CFSE labeled CD34+ cells that homed to the mouse bone marrow of the recipient mice were quantified by FACS.

Even though the same number of cells and CD34+ cells were transplanted from both cultured groups, the homing of nicotinamide-treated CD34+ cells was 6-fold higher, while the homing of CD34+ cells without exposure to nicotinamide was only 2-fold higher relative to the homing of non-cultured CD34+ cells (n=21, p<0.05) (Fig 1a). The homing of cultured cells (MNC) was 2-fold higher with nicotinamide-treated cells compared to nicotinamide-untreated cells, and similar to the homing of non-cultured MNC (n=21, p<0.05) (Fig 1b). Figures 1c-1i show FACS analysis dot plots of representative mice transplanted with non-cultured or cultured cells.

### EXAMPLE 2

### Nicotinamide increases functionality of chemokine receptors and adhesion molecules

Alterations in chemokine and adhesion molecules, either expression or functionality, have been suggested to cause a homing defect in cultured CD34+ cells, since the binding of cells to specific "docking" ligands is critical for the efficient passage of cells from circulation to target tissues (Foguenne, J., et al. Haematologica, 2005;90:445-51). This is specially significant in view of the broad distribution of integrins and adhesion molecules such as VLA-4 and LFA-1 across a variety of cell types (muscle cells, lymphocytes, eosinophils, etc). In order to determine the role of such adhesion and related molecules in nicotinamide-mediated enhancement of homing and engraftment of cells, the effect of nicotinamide on *in-vitro* migration and the functionality of the adhesion molecule Very Late Activation Antigen-4 (VLA-4) was tested.

Using a trans-well migration assay, CXCL12-induced migration of non-cultured and cultured hematopoietic cells was tested, assessing the effects of nicotinamide on integrin and adhesion molecule function. CXCL12 powerfully stimulated the migration of both treated and untreated CD34+ cells (Figure 2d). However, CXCL12-induced migration was significantly higher in cells cultured with nicotinamide (cytokines + NA) compared to the cells cultured without nicotinamide (p>0.02) or non-cultured cells (p=0.05) (Fig. 2). These results suggest that treatment of CD34+ cells with NA can potentially increase the responsiveness of CXCR4 to its ligand CXCL12, resulting in enhanced engraftment and homing potential of the nicotinamide-treated cells.

When the functional quality of cell binding to adhesion molecules was investigated using shear flow analysis, the strong effect of nicotinamide on VLA4-mediated binding and retention on VCAM was revealed. Figure 3 shows the significantly enhanced percentage of initially settled cells resistant to removal by shear stress evident in the cells treated with nicotinamide.

Thus, the results in Figures 2 and 3 reveal that nicotinamide treatment of cells before transplantation increases the function of adhesion and cytokine-related molecules in these cells, enhances cell migration, and therefore enhance cell's transplantation potential, as indicated by increased initial capture and binding to immobilized VCAM-1, and retention under increased flow, as compared to non-cultured or cytokines-alone cultured cells.

### EXAMPLE 4

### NA increased the SCID-repopulating capacity of cytokine-cultured cells

Nicotinamide treatment was tested for ability to enhance homing and engraftment of transplanted cells by repopulation of NOD/SCID mice. To evaluate repopulating capacity, NOD/SCID mice were transplanted with non-cultured CD34⁺ cells (n = 12) over a range of doses intended to achieve a sub-optimal transplantation, and subsequent non-engraftment in a fraction of mice or their progeny following 3-weeks expansion with cytokines (n = 12) or cytokines + NA (n = 13). Human cell engraftment was evaluated 4-weeks post transplantation. Mice were scored as positively engrafted if 0.5% of the recipient bone marrow cells expressed human CD45 antigen (CD45+). As shown in Fig 4a, transplantation of 3 x 10³ CD34⁺ cells resulted in no engraftment in the non-cultured cells. Similarly, the progeny of 3 x 10³ CB CD34⁺ cells cultured with cytokines only also failed to engraft. The presence of nicotinamide in culture, however, resulted in 50% engraftment of 3 x 10³ CB CD34⁺ cells in the mice. At a dose range of 6 x 10³ cells (Fig. 4b), fresh CB CD34⁺ cells engrafted in only 16.7% of the mice, whereas the progeny of 6 x 10³ CD34⁺ cells cultured with cytokines engrafted in 33.3% of the mice. In contrast, at the same dose range, the progeny of cytokines nicotinamide cultured cells engrafted in 100% of the mice (Fig 4b).

The frequency of SCID repopulating cells (SRCs) was calculated using the maximum likelihood estimator as described hereinabove (Figs. 4c-4e). The frequency of SRCs within non-cultured CD34⁺ cells was 1 in 36,756 cells (95% confidence interval [CI], 1/113,366 - 1/11,917) (Fig. 4c.). The SRC frequency within cells cultured with cytokines alone was 1 in 19,982 (CI, 1/47,972 - 1/8,323) (Fig. 4d) and the SRC frequency within cells cultured in the presence of nicotinamide and cytokines was significantly higher, at 1 in 2,620 (CI, 1/5,127 - 1/1,339) (Fig. 4e). Therefore, culture conditions including nicotinamide supported a 14-fold greater number of SRCs than non-cultured cells and 7.6-fold more SRCs than cytokine alone-cultured cells. Fig 4f demonstrates in vivo multilineage differentiation of NA-treated cultured cells engrafted in NOD/SCID mice.

***Effect of nicotinamide on homing and engraftment in IL-3 treated cells:* IL-**3 has been reported to accelerate differentiation and attenuate SCID repopulating ability of transplanted cells. In order to test whether nicotinamide modulates the engraftment potential of cells exposed to the cytokine, nicotinamide's effect on CB-derived CD34+ cells cultured with IL-3 was assessed. Transplantation experiments indicated that treatment with nicotinamide indeed increased the SCID repopulating potential of IL-3 supplemented cultures. Fig. 5a shows the proportion of engraftment following injection of 1.25 to 5 X 10⁴ cells. Figs. 5b-c exhibits the engraftment of total human cells (Fig. 5b) and progenitor cells (Fig. 5c) following transplantation of the lowest cell dose evaluated in this experiment (1.25X10⁴ cells). The results show the presence of human (CD45+) cells in bone marrow of 5 out of 5 mice transplanted with nicotinamide treated cells, but in only 2 out of 5 mice transplanted with cytokine alone treated cells (Fig. 5a). Engraftment of human progenitors (CD45+CD34+) 4-weeks after transplantation was observed only in mice transplanted with cells cultured with nicotinamide.

The results brought hereinabove clearly show that exposure of cells to nicotinamide enhances expression and function of adhesion and integrin molecules critical to cell engraftment and homing, can increase cell migration potential, and clearly provides superior engraftment and homing of transplanted cells. Thus, nicotinamide can be used to provide cell populations for transplantation, having enhanced homing and engraftment potential.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications and GenBank Accession numbers mentioned in this specification are herein incorporated in their entirety by reference into the specification, to the same extent as if each individual publication, patent or patent application or GenBank Accession number was specifically and individually indicated to be incorporated herein by reference. In addition, citation or identification of any reference in this application shall not be construed as an admission that such reference is available as prior art to the present invention.

The teachings of PCT Application IL03/00064, which are hereby incorporated by reference as if fully set forth herein, are intended to be excluded from the scope of the present invention and appending claims.

### LIST OF REFERENCES

Aiuti J. Exp. Med. 1997;185:111-120
Anderlini, P. and Korbling, M. (1997) Stem. Cells 15, 9-17
Baggiolini, M.. 1998, Nature 392:565
Banasik M. et al., J Biol Chem. 1992;267:1569-1575
Bernhard Cancer Res 10: 99, (1995)
Christopherson KW 2nd, et al., Science. 2004 Aug 13;305(5686):1000- 1003
Christopherson KW 2nd, J Immunol. 2002 Dec 15;169(12):7000-7008
Corda D, Di Girolamo M. 2003;22(9):1953-1958
de la Cruz X, Lois S, et al., Bioessays. 2005;27(2):164-75
De Roos et al Transplantation 1997;63:513-18
Fisch Eur J Immunol 26: 595, (1996)
Foguenne, J., et al. Haematologica, 2005;90:445-51
Freedman Nature Medicine 2: 46, (1996)
Gagandeep et al, Gene Therapy 1999;6:729-36
Heslop Nature Medicine 2: 551, (1996)
Humeau L., et al Blood (1997) 90:3496
Ito et al, Muscle Nerve 1998;21:291-7
Imai Br. J. Haematol. 1999;106:905-911
Jaime Imitola et al., Proc Natl Acad Sci U S A. 2004 101(52): 18117-18122
Kipshidze and Serruys, eds. London, UK, 2004
Lupi R, et al., J Biol Chem. 2000;275:9418-9424
Lupi R, et al. Biochem J. 2002:367:1-7
McGrath Dev. Biol. 1999;213:442-456
Protti Cancer Res 56: 1210, (1996)
Rankin PW, et al., J Biol Chem. 1989;264:4312-4317
Roach ML. Methods Mol Biol (2002) 185: 1
Siena Expt Hematol 23:1463, (1996)
Shioda et al. (1998) Proc. Natl. Acad. Sci. USA 95:6331
Smith AG. Annu Rev Cell Dev Biol (2001) 17:435
Smith S. Trends Biochem Sci. 2001;26:174-179
Trounson AO. Reprod Fertil Dev (2001) 13: 523
Ueda K, Hayaishi O, Annu Rev Biochem. 1985;54:73-100
Virág L, Szabó C. Pharm. Reviews. 2002;54:375-429
Yau L, et al., Eur. J. Biochem. 2003;270:101-110

## Claims

1. A method of enhancing cell homing and engraftment potential, the method comprising *ex-vivo* or *in vitro* subjecting a population of cells to an amount of nicotinamide for a period of time sufficient for enhancing cell homing and engraftment potential, the method further **characterized by** at least one of the following:
(i) wherein said amount of nicotinamide and said period of time are selected sufficient to down regulate CD26 expression by cells of said population of cells but not for stem and/or progenitor cell expansion;
(ii) said population of cells does not include, mononuclear cells, early liver progenitor cells, committed progenitor cells, non-hematopoietic stem and progenitor cells, or embryonic stem and progenitor cells;
(iii) said subjecting is in the absence of nutrients;
(iv) said subjecting is in the absence of a cytokine;
(v) said subjecting is in the absence of FLT-3 ligand;
(vii) said subjecting is in the absence of stem cell factor (SCF);
(vii) said subjecting is in the absence of granulocyte colony stimulating factor (GCSF);
(viii) said subjecting is in the absence of an early acting cytokine; and
(ix) said subjecting is in the absence of a late acting cytokine;
and wherein said population of cells does not include a hematopoietic stem and/or progenitor cell population.

2. The method of claim 1, wherein said population of cells is derived from a source selected from the group consisting of umbilical cord blood cells, mobilized peripheral blood cells, bone marrow cells.

3. The method of claim 1, wherein said population of cells is derived from a mononuclear cell fraction.

4. The method of claim 1, wherein said period of time is between 12-30 hours.

5. The method of claim 1, wherein said conditions insufficient for stem cell expansion are selected from the group consisting of absence of nutrients, absence of late acting cytokines and absence of early acting cytokines.

6. The method of claim 1, wherein said period of time is sufficient to downregulate expression of CD26 on the cells, but insufficient for cell proliferation.

7. The method of claim 1, wherein a concentration of said nicotinamide is 0.01-60 mg/ml.

8. The method of claim 1, wherein said effective amount of nicotinamide is 10-20 mg/kg body weight.

9. The method of claim 1, wherein said cells are cultured cells.

10. A method of preparing cells for transplantion in a subject, the method comprising:
(a) ex-vivo subjecting a population of cells comprising the cells to an amount of nicotinamide for a period of time sufficient to enhance homing and engraftment in said cells; the method further **characterized by** at least one of the following:
(i) wherein said amount of nicotinamide and said period of time are selected sufficient to down regulate CD26 expression by cells of said population of cells but not for stem and/or progenitor cell expansion;
(ii) said population of cells does not include hematopoietic cells, hematopoietic stem cells, mononuclear cells, early liver progenitor cells, committed progenitor cells, non-hematopoietic stem and progenitor cells, or embryonic stem and progenitor cells;
(iii) said subjecting is in the absence of nutrients;
(iv) said subjecting is in the absence of a cytokine;
(v) said subjecting is in the absence of FLT-3 ligand;
(vi) said subjecting is in the absence of stem cell factor (SCF);
(vii) said subjecting is in the absence of granulocyte colony stimulating factor (GCSF);
(viii) said subjecting is in the absence of an early acting cytokine;
(ix) said subjecting is in the absence of a late acting cytokine; and subsequently
(b) isolating the cells, wherein said population of cells does not include a hematopoietic stem and/or progenitor cell population.

11. The method of claim 10, wherein said period of time is between 12-30 hours.

12. A cell population comprising the cells **characterized by** enhanced engraftment and homing potential according to the methods of any of claim 1.

13. A pharmaceutical composition comprising as an active ingredient the cell population of claim 12 and a pharmaceutically acceptable carrier.
